(19) Europäisches Patentamt
European Patent Office
Office européen des brevets

(11) EP 1 344 058 B1

(12) **EUROPÄISCHE PATENTSCHRIFT**

(45) Veröffentlichungstag und Bekanntmachung des Hinweises auf die Patenterteilung:
**14.07.2004 Patentblatt 2004/29**

(21) Anmeldenummer: **00990818.7**

(22) Anmeldetag: **22.12.2000**

(51) Int Cl.[7]: **G01N 33/52**, G01N 33/558, G01N 33/543

(86) Internationale Anmeldenummer:
**PCT/EP2000/013187**

(87) Internationale Veröffentlichungsnummer:
**WO 2002/018942 (07.03.2002 Gazette 2002/10)**

(54) **KIT UND VERFAHREN ZUR BESTIMMUNG DES REDOX-STATUS IM URIN**

KIT AND METHOD FOR DETERMINING THE REDOX STATE IN URINE

KIT ET PROCEDE DE DETERMINATION DE L'ETAT REDOX DANS L'URINE

(84) Benannte Vertragsstaaten:
**AT BE CH CY DE DK ES FI FR GB GR IE IT LI LU MC NL PT SE TR**

(43) Veröffentlichungstag der Anmeldung:
**17.09.2003 Patentblatt 2003/38**

(73) Patentinhaber: **Orthomol Pharmazeutische Vertriebs Gmbh**
**40764 Langenfeld (DE)**

(72) Erfinder:
- **GLAGAU, Kristian**
  **40764 Langenfeld (DE)**
- **HAUCK, Ralf-Siegbert**
  **40721 Hilden (DE)**

(74) Vertreter: **Albrecht, Thomas, Dr.**
**Kraus & Weisert,**
**Thomas-Wimmer-Ring 15**
**80539 München (DE)**

(56) Entgegenhaltungen:

**US-A- 4 148 611** **US-A- 4 670 385**
**US-A- 5 726 063** **US-A- 5 958 714**

- **DATABASE WPI Section Ch, Week 198218 Derwent Publications Ltd., London, GB; Class B04, AN 1982-35814E XP002180841 & JP 57 049862 A (DAIICHI KAGAKU YAK), 24. März 1982 (1982-03-24)**

Bemerkungen:

Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft ein Kit und ein Verfahren zur Bestimmung des Redox-Status im Urin. Insbesondere betrifft die Erfindung ein solches Kit und ein solches Verfahren, bei dem die Bestimmung des Redox-Status in besonders einfacher Weise aufgrund der Farbreaktion mit einem Reagenz ausgewählt aus der Gruppe der Indolderivate durch visuellen Farbvergleich, d.h. mit bloßem Auge, durchgeführt werden kann.

## Hintergrund der Erfindung

Der menschliche Organismus ist insbesondere unter den heutigen Umweltbedingungen einer großen Zahl von Belastungen ausgesetzt, durch die in den Zellen des Körpers freie Radikale erzeugt werden. Zu diesen Belastungen gehören z.B. Zigarettenrauch, Autoabgase, UV-Strahlung und verschiedene andere Noxen. Die erzeugten freien Radikale, zu denen z.B. auch freie Sauerstoffradikale gehören, erzeugen in vielfältigen Folgereaktionen pathogene Abbauprodukte, die verschiedene Bestandteile der Körperzelle nachhaltig schädigen können. Die Folgen dieser Schädigungen sind z.B. vorzeitiges Altern, aber auch ernste Erkrankungen wie etwa Diabetes oder Krebs.

Zur Unschädlichmachung, d.h. Bindung freier Radikale, die auch unter idealen Umweltbedingungen im begrenzten Maße auftreten, hat der Körper des Menschen eine Reihe von anti-oxidativ wirkenden Systemen entwickelt. Diese Systeme sind jedoch häufig, u.a. in Abhängigkeit von den Lebensgewohnheiten und der speziellen Umweltsituation eines Menschen, überlastet, so dass freie Radikale im Körper nicht im ausreichenden Maße unschädlich gemacht werden können. In solchen Fällen ist es notwendig, die körpereigenen anti-oxidativen Systeme durch die Einnahme spezieller Nahrungsergänzungen (Mikronährstoffzubereitungen aus Vitaminen und Spurenelementen) zu stimulieren bzw. zu unterstützen. Bei einer solchen Behandlung sollte die Menge der verabreichten Medikamente bzw. eingenommenen Ergänzungsstoffe an die tatsächliche Belastung des Körpers mit freien Radikalen angepasst werden.

Idealerweise soll die Bestimmung der Belastung durch freie Radikale von dem jeweiligen Patienten.selber ohne großen Aufwand schnell und kostengünstig durchgeführt werden können. Eine solche Bestimmung kann deshalb insbesondere anhand von Urinproben durchgeführt werden.

Das US-Patent 5,726,063 beschreibt in 2-Position arylsubstituierte Indole zur kolorimetrischen Bestimmung von Malondialdehyd und anderen Enaldehyden als Lipidperoxidationsindizes in wässrigen Medien.

## Beschreibung der Erfindung

Der vorliegenden Erfindung liegt demgemäss die Aufgabe zugrunde, den Redox-Status im Urin in einfacher Weise, d.h. mit dem bloßen Auge, zu bestimmen. Unter "Bestimmung des Redox-Status" ist in diesem Zusammenhang die Gewinnung einer quantitativen, halbquantitativen oder qualitativen Aussage über die Konzentration bzw. das Vorhandensein von freien Radikalen im Körper des Menschen, d.h. über die oxidative Belastung des Organismus zu verstehen. Als eine Indikatorsubstanz für diese Aussage dient der Malondialdehyd im Urin, wobei ggf. jedoch auch andere relevante Substanzen erfasst werden können.

Diese Aufgabe wird erfindungsgemäß gelöst durch ein Kit zur Bestimmung des Redox-Status im Urin, umfassend:

(a) mindestens ein Reagenz ausgewählt aus der Gruppe der Verbindungen der allgemeinen Formel (I)

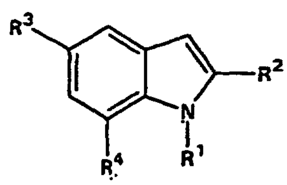

(I)

worin $R_1$ für H oder $C_1$-$C_{10}$-Alkyl steht, und

$R_2$ für $C_1$-$C_{10}$-Alkyl steht, und $R_3$ und $R_4$, unabhängig voneinander, für H, $C_1$-$C_{10}$-Alkyl, $C_6$-$C_{14}$-Aryl, $NH_2$, NHR, $NR_2$, NHCOR, OH, OR, OCOR, SH, SR, F, Cl, Br, $CF_3$ oder $CCl_3$ stehen,

wobei Arylgruppen ggf. substituiert sind mit einem oder mehreren Substituenten ausgewählt aus der Gruppe bestehend aus $C_1$-$C_6$-Alkyl, $NH_2$, NHR, $NR_2$, NHCOR, OH, OR, OCOR, SH, SR, F, Cl, Br, $CF_3$ und $CCl_3$ und

wobei R jeweils für $C_1$-$C_6$-Alkyl steht und mehrere R-Gruppen ggf. unabhängig voneinander ausgewählt werden können,

(b) mindestens eine Säure und

(c) eine Farbskala zum visuellen Farbvergleich, wobei den einzelnen Farben der Skala ein bestimmter Redox-Status des zu untersuchenden Urins zugeordnet ist.

Gemäß einer weiteren Ausführungsform der Erfindung wird auch ein Verfahren zur Bestimmung des Redox-Status im Urin bereitgestellt, das die folgenden Stufen umfasst:

(a) Vermischen einer zu untersuchenden Urinprobe mit

(i) mindestens einem Reagenz ausgewählt aus der Gruppe der Verbindungen der allgemeinen Formel (I)

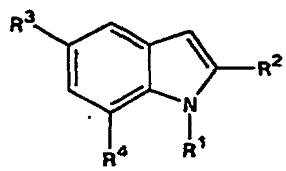

(I)

worin $R_1$ für H oder $C_1$-$C_{10}$-Alkyl steht, und

$R_2$ für $C_1$-$C_{10}$-Alkyl steht, und $R_3$ und $R_4$, unabhängig voneinander, für H, $C_1$-$C_{10}$-Alkyl, $C_6$-$C_{14}$-Aryl, $NH_2$, NHR, $NR_2$, NHCOR, OH, OR, OCOR, SH, SR, F, Cl, Br, $CF_3$ oder $CCl_3$ stehen, wobei Arylgruppen ggf. substituiert sind mit einem oder mehreren Substituenten ausgewählt aus der Gruppe bestehend aus $C_1$-$C_6$-Alkyl, $NH_2$, NHR, $NR_2$, NHCOR, OH, OR, OCOR, SH, SR, F, Cl, Br, $CF_3$ und $CCl_3$ und

wobei R jeweils für $C_1$-$C_6$-Alkyl steht und mehrere R-Gruppen ggf. unabhängig voneinander ausgewählt werden können, und

(ii) mindestens einer Säure und

(b) nachdem sich eine stabile Färbung der Probe eingestellt hat, visuelles Vergleichen der Färbung der Probe mit einer Farbskala, wobei den einzelnen Farben der Skala ein bestimmter Redox-Status des zu untersuchenden Urins zugeordnet ist.

Das erfindungsgemäße Kit und das erfindungsgemäße Verfahren beruhen auf einer Farbreaktion, die das Reagenz der Formel (I) unter sauren Bedingungen bei Vermischen mit der zu untersuchenden Urinprobe zeigt. Diese Farbreaktion führt zur Bildung eines für das Ausgangsreagenz charakteristisch gefärbten Farbstoffes, wobei die Intensität der Färbung von der oxidativen Belastung des Organismus abhängt. Dabei ist die Färbung um so intensiver, je stärker diese Belastung, d.h. je höher die Konzentration freier Radikale im Körper ist. Im allgemeinen wird die mit dem bloßen Auge wahrnehmbare Farbe der Probe nach Ausbildung des Farbstoffes auch durch die Ausgangsfarbe des untersuchten Urins beeinflusst.

Diese Farbreaktion wird insbesondere ausgelöst durch das Vorhandensein von Malondialdehyd im untersuchten Urin. Malondialdehyd ist ein Zerfallsprodukt bestimmter Hydroperoxide, die durch die Oxidation ungesättigter Fettsäuren der Zellmembran im Organismus gebildet werden. Da oxidativer Stress zu einer verstärkten Lipidoxidation der Zelle führt, bedingt dieser auch einen Anstieg der Konzentration von Malondialdehyd im Urin. Dieser Anstieg kann mit Hilfe des erfindungsgemäßen Kits und durch das erfindungsgemäße Verfahren festgestellt werden, so dass dann insbesondere in Abhängigkeit von der Stärke der Belastung Gegenmaßnahmen getroffen werden können, wie z.B. Stärkung der anti-oxidativen Abwehr durch Einnahme von geeigneten Vitaminen und Spurenelementen und/oder Veränderung der Lebensweise (Rauchen, Ernährung, Wohn-/Arbeitsumfeld).

Die Bestimmung des Redox-Status mit Hilfe des erfindungsgemäßen Kits bzw. durch das erfindungsgemäße Verfahren erfolgt, indem das Reagenz, die Säure und die Urinprobe bei Raumtemperatur miteinander vermischt werden, woraufhin sich die insbesondere von der Stärke der oxidativen Belastung abhängige Färbung einstellt. Die Farbreaktion ist im allgemeinen innerhalb von 15 bis 60 Minuten, bei bevorzugten Ausführungsformen innerhalb von 15 bis 30 Minuten abgeschlossen. Danach, d.h. sobald die Intensität der Färbung nicht mehr zunimmt, wird diese Intensität durch Vergleich mit einer Farbskala visuell, d.h. mit bloßem Auge, festgestellt. Dabei besteht die Farbskala aus einer Anzahl diskreter Farbelemente, die entsprechend ihrer Intensität angeordnet sind und deren Farben der Färbung der Probelösung nach Abschluss der Farbreaktion je nach Redox-Status entsprechen.

## Kurze Beschreibung der Zeichnungen

Die Figuren 1 bis 3 zeigen bevorzugte Ausführungsformen erfindungsgemäß verwendbarer Zwei- bzw. Mehrkammersysteme.

Die Figuren 4 bis 6 zeigen bevorzugte Ausführungsformen erfindungsgemäß verwendbarer Teststreifen.

**Beschreibung bevorzugter Ausführungsformen**

Bei den Reagenzien der allgemeinen Formel (I) handelt es sich um Indolderivate. In der Formel (I) können $R^1$, $R^2$, $R^3$ und $R^4$ unter anderem für eine Alkylgruppe mit 1 bis 10 Kohlenstoffatomen stehen. Beispiele für solche Alkylgruppen sind Methyl, Ethyl, n-Propyl, i-Propyl, n-Butyl, sec-Butyl, iso-Butyl, tert-Butyl, Pentyl und Hexyl. Bevorzugt werden Alkylgruppen mit 1 bis 6 Kohlenstoffatomen und besonders bevorzugt solche mit 1 bis 4 Kohlenstoffatomen. Eine besonders bevorzugte Alkylgruppe ist Methyl.

In der Formel (I) können $R^3$ und $R^4$ unter anderem auch für eine Arylgruppe mit 6 bis 14, bevorzugt 6 bis 10 Kohlenstoffatomen stehen. Diese Arylgruppe kann ggf. mit einem oder mehreren Substituenten ausgewählt aus der Gruppe bestehend aus $C_1$-$C_6$-Alkyl, $NH_2$, NHR, $NR_2$, NHCOR, OH, OR, OCOR, SH, SR, F, Cl, Br, $CF_3$ und $CCl_3$ substituiert sein wobei R jeweils für $C_1$-$C_6$-Alkyl steht und mehrere R-Gruppen ggf. unabhängig voneinander ausgewählt werden können. Eine besonders bevorzugte Arylgruppe ist Phenyl.

In der Formel (I) können $R^3$ und $R^4$ auch für $NH_2$, NHR, $NR_2$, NHCOR, OH, OR, OCOR, SH, SR, F, Cl, Br, $CF_3$ oder $CCl_3$ stehen, wobei R jeweils für $C_1$-$C_6$-Alkyl steht und mehrere R-Gruppen ggf. unabhängig voneinander ausgewählt werden können. Bei diesen Gruppen handelt es sich insbesondere um Substituenten mit positivem mesomeren Effekt. Eine besonders bevorzugte Gruppe dieser Art ist Methoxy.

Bevorzugte Reagenzien der allgemeinen Formel (I) sind: 2-Methylindol und 1,2-Dimethylindol.
Diese Reagenzien werden insbesondere im Hinblick auf ihre Stabilität bzw. die Langzeit-Lagerstabilität ihrer Lösungen und/oder im Hinblick auf ihre Verfügbarkeit in (hoch)reiner Form bevorzugt.

Erfindungsgemäß wird eine Säure verwendet, wobei es sich um eine anorganische oder eine organische Säure handeln kann. Geeignete Säuren sind z.B. Salzsäure, Schwefelsäure, Phosphorsäure, Methansulfonsäure, Citronensäure, Oxalsäure, Trifluoressigsäure oder Trichloressigsäure. Besonders bevorzugt wird Salzsäure eingesetzt.

Das Reagenz und/oder die Säure können in Form einer Lösung in einem geeigneten Lösungsmittel eingesetzt werden. Geeignet sind prinzipiell alle Lösungsmittel, die mit Wasser mischbar sind und nicht stark toxisch wirken. Geeignet sind insbesondere niedere Alkohole, wie Methanol, Ethanol, Propanol, Isopropanol oder Butanol, aber in geschlossenen Systemen etwa auch Acetonitril, Tetrahydrofuran oder Hexamethylphosphorsäuretriamid. Bevorzugt sind Ethanol und Isopropanol, von denen letzteres besonders bevorzugt ist.

Wenn das Reagenz in Form einer Lösung eingesetzt wird, beträgt dessen Konzentration darin im allgemeinen 0,1 bis 50 mmol/l, bevorzugt 5 bis 15 mmol/l, besonders bevorzugt 7 bis 12 mmol/l.

Wenn die Säure in Form einer Lösung eingesetzt wird, beträgt deren Konzentration darin im allgemeinen 0,1 bis 5 mol/l, bevorzugt 0,5 bis 1,5 mol/l, besonders bevorzugt 0,9 bis 1,2 mol/l.

Bevorzugt wird als Säure eine Lösung von Salzsäure in Methanol, Ethanol oder Isopropanol eingesetzt, wobei eine Lösung von Salzsäure in Isopropanol besonders bevorzugt ist.

Da die Färbung der untersuchten Urinprobe im allgemeinen auch von der Farbe des zu untersuchenden Urins, d.h. dessen mehr oder weniger starker Gelbfärbung, abhängt, ist es zweckmäßig, verschiedene Farbskalen für die jeweiligen Ausgangsurinfarben bereitzustellen. Diese Skalen können insbesondere in Form einer Farbmatrix angeordnet sein, wobei die erste Zeile der Matrix, die in Frage kommenden Urinfarben enthält, deren Übereinstimmung zunächst durch Vergleich mit dem unbehandelten Urin festgestellt wird. Der Farbvergleich mit der Probe nach Beendigung der Farbreaktion wird dann innerhalb derjenigen Spalte der Farbmatrix durchgeführt, die durch die Übereinstimmung der Farbe des Ausgangsurins festgestellt wurde.

Im Hinblick auf den Aufwand zur Durchführung der Bestimmung des Redox-Status mit Hilfe des erfindungsgemäßen Kits bzw. durch das erfindungsgemäße Verfahren werden die dazu notwendigen Komponenten, d.h. das Reagenz und die Säure, in solcher Form bereitgestellt bzw. eingesetzt, dass deren Portionierung bzw. Handhabung soweit wie möglich vereinfacht werden. In diesem Zusammenhang ist allerdings zu beachten, dass Lösungen des Reagenzes unter sauren Bedingungen eine geringe Lagerstabilität aufweisen, so dass das Reagenz und die Säure erst kurz vor Durchführung der eigentlichen Bestimmung miteinander in Kontakt gebracht werden können.

Gemäß einer besonderen Ausführungsform der Erfindung wird deshalb das Reagenz oder eine Lösung davon in einem Behälter eingeschlossen und dadurch von der Säure getrennt, wobei dieser Behälter durch vorsätzliche mechanische Einwirkung leicht zerstört oder geöffnet werden kann und so angeordnet ist, dass das Reagenz durch die Zerstörung oder die Öffnung des Behälters unmittelbar in Kontakt mit der Säure und/oder mit einer Lösung davon und/oder mit der Urinprobe tritt. Bei dem genannten Behälter kann es sich z.B. um eine Glasampulle handeln. Diese Glasampulle kann ihrerseits innerhalb eines Gefäßes angeordnet sein, das die Säure bzw. eine Lösung davon enthält, und zu dem zur Durchführung der Bestimmung die Urinprobe gegeben wird. Indem die Glasampulle innerhalb des Gefäßes zerbrochen wird, so dass sich der Inhalt der Ampulle und des Gefäßes vermischen, kann dann die Farbreaktion ausgelöst werden.

Entsprechend kann auch statt des Reagenzes die Säure in einem separaten Behälter eingeschlossen werden, der wie oben beschrieben angeordnet ist und entsprechend geöffnet bzw. zerstört wird.

Eine weitere Ausführungsform eines erfindungsgemäß verwendbaren Zweikammersystems ist schematisch in Figur 1 dargestellt. Das darin dargestellte System besteht aus zwei Gefäßen 11 und 16, die jeweils mit Schraubgewinden 12 bzw. 14 ausgestattet sind, über die sie miteinander verbunden werden können. Bei diesem Verbinden durchsticht eine spitz ausgeformte Öffnung 13 des Gefäßes 11 eine Membran 15, mit der das Gefäß 16 verschlossen ist. In dem Gefäß 16 befindet sich die eine Reaktionskomponente 17 (Reagenz oder Säure), während die andere Komponente (Säure bzw. Reagenz) in dem Gefäß 11 enthalten ist und erst nach durchstoßen der Membran 15 in das Gefäß 16 gelangt. Vorteilhafterweise kann das Gefäß 11 als Kunststoffflasche ausgestaltet sein, die zusammengedrückt werden kann, um ihren Inhalt in das Gefäß 16 zu überführen. Die zu untersuchende Urinprobe kann dabei auch in das Gefäß 11 aufgesaugt werden, bevor sie zusammen mit der einen Reaktionskomponente in das Gefäß 16 überführt wird.

Ein erfindungsgemäß verwendbares Zweikammersystem kann auch wie in Figur 2 schematisch gezeigt ausgestaltet sein. Das darin gezeigte System besteht aus zwei Gefäßen 23 und 27, die mit Schraubgewinden 25 und 26 versehen sind und dadurch dicht miteinander verbunden werden können. In dem Gefäß 23 ist durch eine Membran 24 eine' Kammer 22 gebildet, die eine der Reaktionskomponenten (Reagenz oder Säure) enthält. Durch Eindrücken des Stiftes 21 wird die Membran 24 durchstoßen, so dass die in der Kammer 22 enthaltene Reaktionskomponente mit der anderen, in dem Gefäß 27 enthaltenen Komponente 28 (Säure bzw. Reagenz) in Kontakt kommt. Die zu untersuchende Urinprobe kann vorteilhafterweise vor dem Durchstoßen der Membran 24, ggf. aber auch nach dem Vermischen von Reagenz und Säure, in das Gefäß 27 eingebracht werden.

In einer Abwandlung des in Figur 2 gezeigten Systems kann der Stift 21 auch weggelassen werden, wenn die Gewinde und die Membran so ausgestaltet werden, dass die Membran beim Zusammenschrauben der Gefäße zerrissen wird.

Eine weitere vorteilhafte Variante eines erfindungsgemäß verwendbaren Mehrkammersystems ist in Figur 3 gezeigt. Auch dieses System besteht aus zwei Gefäßen 34 und 37, die durch Schraubgewinde 35 und 36 miteinander verbunden werden können. Das Reagenz und die Säure sind hier in zwei in Verbindung mit dem Gefäß 34 ausgebildeten Kammern 32 und 33 enthalten und werden durch Betätigen des Stempels 31 miteinander in Kontakt gebrach. Bei diesem System kann das Gefäß 37 vorteilhafterweise als unmittelbar zur Aufnahme der Urinprobe dienender Becher ausgebildet sein.

Im Hinblick auf die Dosierung und Handhabung des Reagenz kann dieses vorteilhafterweise außer in reiner Form auch in Form einer Verreibung mit einem neutralen Trägerstoff als Pulver, Tablette oder auf einem festen Träger (z.B. einem Kunststoffstreifen) vorliegen. Als neutrale Trägerstoffe kommen z.B. in Betracht: neutrale Alkalisalze, wie z.B. Natriumchlorid, oder inerte Träger höherer Dichte, wie z.B. $SiO_2$. Das Gewichtsverhältnis von Reagenz zu Trägerstoff beträgt dabei im allgemeinen 1 : 0 (reines Reagenz) bis 1 : 1000, bevorzugt 1 : 0 bis 1 : 200.

Um die Durchführung der Bestimmung des Redox-Status zu erleichtern, kann das erfindungsgemäße Kit ein Gefäß zur unmittelbaren Aufnahme des ausgeschiedenen Urins enthalten. Dieses Gefäß kann z.B. in Form eines einfachen Bechers ausgestaltet sein. Des weiteren kann das erfindungsgemäße Kit z.B. ein Pipettiermittel enthalten. Dieses kann insbesondere dazu dienen, eine abgemessene Menge des zu untersuchenden Urins aus dem zuvor erwähnten Gefäß zur Probenahme in dasjenige Gefäß zu überführen, in dem die Farbreaktion durchgeführt wird. Als Pipettiermittel können insbesondere handelsübliche Einwegpipetten verwendet werden.

Des weiteren kann das erfindungsgemäße Kit ein Analysegefäß enthalten, in dem die Farbreaktion durch Vermischen des Reagenz, der Säure und der Urinprobe durchgeführt wird. Gemäß einer besonders vorteilhaften Ausführungsform kann die Farbskala so mit dem Analysegefäß verbunden sein, dass sich die Probelösung und die einzelnen Farbelemente der Skala in unmittelbarer räumlicher Nähe befinden, so dass der Farbvergleich unmittelbar, d. h. auf einen Blick, durchgeführt werden kann.

Gemäß einer weiteren vorteilhaften Ausführungsform der Erfindung sind das Reagenz und die Säure beide in fester Form in einen Teststreifen eingearbeitet, der unmittelbar in die zu untersuchende Probe eingetaucht werden kann. In diesem Fall wird als Säure bevorzugt Citronensäure, Oxalsäure oder Trichloressigsäure eingesetzt.

Eine einfache Ausführungsform eines solchen Teststreifens ist in Figur 4 schematisch dargestellt. Bei dem darin gezeigten Teststreifen 44 ist die Säure in zwei diskreten Bereichen 41 und 43 aufgebracht, zwischen denen sich ein Bereich 42 befindet, der das Reagenz enthält. Wenn der Teststreifen in die zu untersuchende Urinprobe eingetaucht wird, diffundiert die Säure in den Reagenzbereich 42, wo die Farbrektion eintritt. Nach deren Beendigung wird die Farbe dieses Bereichs mit der Farbskala verglichen.

Ein erfindungsgemäß verwendbarer Teststreifen kann auch in Form einer in Figur 5 schematisch gezeigten Test-Kassette ausgestaltet sein. Die Kassette 52 enthält einen Vliesstoffstreifen, auf den im Bereich der Öffnung 53 die Urinprobe aufgetragen wird. Die Probe breitet sich dann durch den Streifen in der durch den Pfeil 51 angegebenen Richtung aus. In dem Bereich 54 ist die Säure aufgebracht. Diese wird von der Lösung auf dem Weg zu dem das Reagenz enthaltenden Bereich 55 aufgenommen, so dass die Farbreaktion in dem Bereich 55 eintritt. Der Farbvergleich kann dann durch eine entsprechende Öffnung hindurch vorgenommen werden.

Ein Teststreifen kann erfindungsgemäß auch wie in Figur 6 schematisch gezeigt ausgestaltet sein bzw. verwendet werden. Hier befindet sich die Säure in dem Bereich 63 und das Reagenz in dem Bereich 62 des Streifens 61. Der Streifen 61 wird in die zu untersuchende Urinprobe 65, die sich am Boden des Gefäßes 64 befindet, eingetaucht. Daraufhin breitet sich die Probelösung durch Kapillarkräfte nach oben hin aus. Dabei passiert sie den Bereich 63, wo sie die Säure aufnimmt, bevor im Bereich 62 die Farbrektion eintritt.

**Beispiele**

Die vorliegende Erfindung wird anhand der folgenden Beispiele näher erläutert.

**Beispiel 1** (Bestimmung des Redox-Status)

Nach drei verschiedenen Varianten werden die in der folgenden Tabelle 1 jeweils angegebenen Komponenten (Reagenz und Säure) in den in der Tabelle 1 ebenfalls angegebenen Mengen mit jeweils 1 ml frischem Morgenurin versetzt. Das so erhaltene Gemisch wird in einem verschlossenen Gefäß kräftig geschüttelt und stehen gelassen. Nach 15 bis 20 Minuten wird die entstandene Färbung mit einer Farbskala verglichen.

Tabelle 1

| Variante | Komponente | Zusammensetzung | Menge |
|---|---|---|---|
| 1 | Reagenz | 100 mg 2-Methylindol gelöst in 100 ml Isopropanol | 1 ml |
| | Säure | 25%-ige wässrige HCl-Lösung | 1 ml |
| 2 | Reagenz | 100 mg 2-Methylindol gelöst in 50 ml Isopropanol | 1 ml |
| | Säure | 25 ml 25%-ige wässrige HCl-Lösung vermischt mit 50 ml Isopropanol | 1 ml |
| 3 | Reagenz | 100 mg 2-Methylindol verrieben mit 20 g Natriumchlorid | 0,20 g |
| | Säure | 25 ml 25%-ige wässrige HCl-Lösung vermischt mit 100 ml Isopropanol | 2 ml |

**Beispiel 2** (Untersuchung des Verhaltens spezieller Reagenzien)

In diesem Beispiel wurde das Verhalten der folgenden Reagenzien A bis E untersucht (wobei die Reagenzien C bis E nicht zu den erfindungsgemäß verwendbaren Reagenzien gehören) :

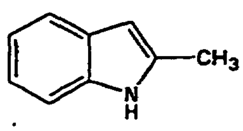
A

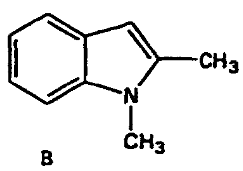
B

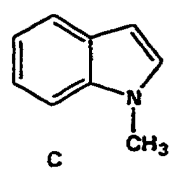
C

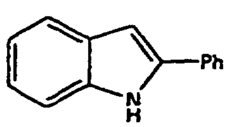
D

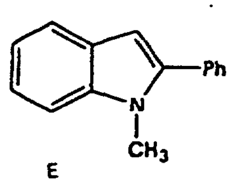
E

Als "Modell-Urin" wurden dabei wässrige Lösungen von Malondialdehyd verwendet, und zwar mit Konzentrationen von 10 µmol/l bzw. 1,0 µmol/l. (Typische Malondialdehydkonzentrationen im menschlichen Urin betragen größenordnungsmäßig 0,5 bis 5 µmol/l.)

Als Reagenzlösung diente eine Lösung von 100 mg Reagenz (A bis E) gelöst in 50 ml Isopropanol. Als Säurelösung diente eine Gemisch aus 25 ml 25%-iger wässriger HCl-Lösung und 50 ml Isopropanol.

Die Untersuchung erfolgte, indem jeweils 1 ml der Testlösung mit 1 ml Reagenzlösung und 1 ml Säurelösung versetzt wurde. Das so erhaltene Gemisch wurde in einem verschlossenen Gefäß kräftig geschüttelt und bei 22 °C stehen gelassen. Die eingetretenen Färbungen wurden dann jeweils einmal nach 15 bis 20 Minuten und ein weiteres Mal nach 60 Minuten bestimmt und sind in der folgenden Tabelle 2 angegeben.

Die einzelnen Färbungen wurden dabei jeweils durch Vergleich mit den HKS™-Standard-Farbtabellen bzw. -fächern (Rasterfächer "K", Ausgabe 1993, Fa. Hostmann-Steinberg, Celle, Vertrieb durch Fa. HKVdruck GmbH, Stuttgart) bestimmt, wobei in Tabelle 2 jeweils der Farbcode (z.B. "45K") und die Sättigung bzw. Helligkeitsstufe (z.B. 60%) bei einer Graustufe von 0% angegeben ist.

Die Reagenzien A und B ergeben rote Farbtöne, Reagenz C ergibt einen blau-grauen Farbton und die Reagenzien D und E ergeben blaue Farbtöne.

Tabelle 2

| Reagenz | Malondialdehydkonzentration | | | |
|---|---|---|---|---|
| | 10 μmol/l | | 1,0 μmol/l | |
| | Reaktionszeit | | Reaktionszeit | |
| | 15 bis 20 min | 60 min | 15 bis 20 min | 60 min |
| A | 27K (100%) | 27K (100%) | 27K (100%) | 27K (100%) |
| B | 31K/29K* (100%) | 31K/29K* (100%) | 27K/33K* (100%) | 27K/33K* (100%) |
| C | 36K (100%) | 82K (40%) | 28K (100%) | 1K (60%) |
| D | 45K (60%) | 45K (100%) | 26K (20%) | 34K (60%) |
| E | 40K (80%) | 43K (100%) | 40K (10%) | 40K (50%) |

*Bei diesen Proben lag der Farbton zwischen zwei Farben der Vergleichsskala

Anhand dieser Untersuchungen wurden folgende Abstufungen der Reaktionsgeschwindigkeit (jeweils in abnehmender Reihenfolge) der einzelnen Reagenzien festgestellt:

(a) bei einer Malondialdehydkonzentration von 10 μmol/l:

A = B = C > D > E

(b) bei einer Malondialdehydkonzentration von 1,0 μmol/l:

A > B > C >> D > E

In einer analogen Untersuchung mit Variation der verwendeten Säure (entsprechend Beispiel 1, Variante 2; Malondialdehydkonzentration = 10 μmol/l) wurde folgende Abstufung der Reaktionsgeschwindigkeit (in abnehmender Reihenfolge) festgestellt: Salzsäure >> Trichloressigsäure > Oxalsäure > Citronensäure.

**Beispiel 3** (Verfolgung des Redox-Status bei Verabreichung anti-oxidativ wirkender Mikronährstoffe)

Ausgehend von oxidativ unbelasteten Urinproben mit verschieden starken Gelbfärbungen (entsprechend den HKS™-Referenzfarben 1K, 3K und 5K; vgl. die Angaben bei Beispiel 2) und mit Hilfe von Malondialdehyd-Verdünnungsreihen wurde für jede Ausgangs-Urinfarbe eine Farbskala erstellt, deren.Abstufungen im folgenden mit I (geringe oxidative Belastung) bis III (starke oxidative Belastung) bezeichnet werden.

Zunächst wurden Morgenurinproben von 61 freiwilligen, gesunden Probanden auf deren Redox-Status untersucht, wobei im einzelnen wie in Beispiel 1, Variante 2 vorgegangen wurde. Die Untersuchung erfolgte am gleichen Vormittag der Probenahme. Dabei ergab sich folgende Häufigkeit der einzelnen Belastungsstufen:

I: 41 Probanden
II: 15 Probanden
III: 5 Probanden

Außerdem wurden die Proben mit Hilfe entsprechender, handelsüblicher Teststreifen auf ihren pH-Wert und ihren Gehalt an Ascorbinsäure untersucht, wobei keinerlei Korrelation zwischen diesen Werten und dem erfindungsgemäß bestimmten Redox-Status festgestellt werden konnte.

Drei Probanden, deren Proben bei der ersten Untersuchung eine starke oxidative Belastung (Stufe III) aufwiesen, wurde dann noch am selben Tag (Tag 1), nach der ersten Urinprobenahme, eine Dosis eines handelsüblichen Mikronährstoffpräparats mit folgenden anti-oxidativ wirkenden Bestandteilen:

| | |
|---|---|
| Vitamin A | 2500 I.E. = 0,75 g |
| Vitamin C | 950 mg |
| Vitamin E | 150 mg |
| Beta-Carotin | 15 mg |

und folgenden für die anti-oxidative Wirkung verschiedener Enzyme notwendigen Spurenelementen:

| | |
|---|---|
| Selen | 50 µg |
| Eisen | 0,8 mg |
| Zink | 10 mg |
| Mangan | 2 mg |
| Kupfer | 0,5 mg |

verabreicht.

Jeweils am Morgen der folgenden Tage (Tag 2 bis Tag 4) wurden den drei Probanden wiederum Urinproben entnommen und wie am ersten Tag untersucht, wobei allerdings die Gabe des genannten Mikronährstoffpräparats an den Tagen 2 bis 4 nicht wiederholt wurde. Die Ergebnisse der Untersuchungen dieser Urinproben sind in der folgenden Tabelle 3 angegeben.

Tabelle 3

| Proband | Tag 1 | Tag 2 | Tag 3 | Tag 4 |
|---|---|---|---|---|
| 1 | III | I | II | III |
| 2 | III | I | II | II |
| 3 | III | II | III | III |

Diese Untersuchung, insbesondere die am ersten Tag nach der Gabe des anti-oxidativ wirkenden Präparats (Tag 2) festgestellte, geringe Belastung, zeigt deutlich, dass das erfindungsgemäße Kit bzw. das erfindungsgemäße Verfahren zur Beurteilung des Redox-Status geeignet sind.

**Patentansprüche**

1. Kit zur Bestimmung des Redox-Status im Urin umfassend:

(a) mindestens ein Reagenz ausgewählt aus der Gruppe der Verbindungen der allgemeinen Formel (I)

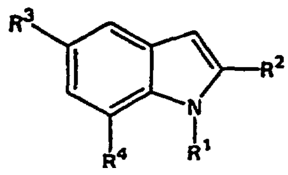

(I)

worin $R^1$ für H oder $C_1$-$C_{10}$-Alkyl steht, und $R^2$ für $C_1$-$C_{10}$-Alkyl steht, und $R^3$ und $R^4$, unabhängig voneinander, für H, $C_1$-$C_{10}$-Alkyl, $C_6$-$C_{14}$-Aryl, $NH_2$, NHR, $NR_2$, NHCOR, OH, OR, OCOR, SH, SR, F, Cl, Br, $CF_3$ oder $CCl_3$ stehen, wobei Arylgruppen ggf. substituiert sind mit einem oder mehreren Substituenten ausgewählt aus der Gruppe bestehend aus $C_1$-$C_6$-Alkyl, $NH_2$, NHR, $NR_2$, NHCOR, OH, OR, OCOR, SH, SR, F, Cl, Br,

$CF_3$ und $CCl_3$ und
wobei R jeweils für $C_1$-$C_6$-Alkyl steht und mehrere R-Gruppen ggf. unabhängig voneinander ausgewählt werden können,
(b) mindestens eine Säure und
(c) eine Farbskala zum visuellen Farbvergleich, wobei den einzelnen Farben der Skala ein bestimmter Redox-Status des zu untersuchenden Urins zugeordnet ist.

**2.** Kit nach Anspruch 1, **dadurch gekennzeichnet, dass** das Reagenz 2-Methylindol oder 1,2-Dimethylindol ist.

**3.** Kit nach Anspruch 1 oder 2, **dadurch gekennzeichnet, dass** die Säure Salzsäure ist.

**4.** Kit nach einem der Ansprüche 1 bis 3, **dadurch gekennzeichnet, dass** das Reagenz in Form einer Lösung in mindestens einem Lösungsmittel vorliegt.

**5.** Kit nach Anspruch 4, **dadurch gekennzeichnet, dass** die Konzentration des Reagenz in der Lösung 0,1 bis 50 mmol/l beträgt.

**6.** Kit nach einem der Ansprüche 1 bis 5, **dadurch gekennzeichnet, dass** die Säure in Form einer Lösung in mindestens einem Lösungsmittel vorliegt.

**7.** Kit nach Anspruch 6, **dadurch gekennzeichnet, dass** die Konzentration der Säure in der Lösung 0,1 bis 5,0 mol/l beträgt.

**8.** Kit nach einem der Ansprüche 4 bis 7, **dadurch gekennzeichnet, dass** das Lösungsmittel Isopropanol umfasst.

**9.** Kit nach einem der Ansprüche 1 bis 8, **dadurch gekennzeichnet, dass** das Reagenz oder eine Lösung davon in einem verschlossenen Behälter enthalten und von der Säure getrennt ist, der durch vorsätzliche mechanische Einwirkung leicht zerstört oder geöffnet werden kann und so angeordnet ist, dass das Reagenz durch die Zerstörung oder Öffnung des Behälters unmittelbar in Kontakt mit der Säure und/oder mit einer Lösung davon und/oder mit der Urinprobe tritt.

**10.** Kit nach einem der Ansprüche 1 bis 8, **dadurch gekennzeichnet, dass** die Säure oder eine Lösung davon in einem verschlossenen Behälter enthalten und von dem Reagenz getrennt ist, der durch vorsätzliche mechanische Einwirkung leicht zerstört oder geöffnet werden kann und so angeordnet ist, dass die Säure durch die Zerstörung oder Öffnung des Behälters unmittelbar in Kontakt mit dem Reagenz und/oder mit einer Lösung davon und/oder mit der Urinprobe tritt.

**11.** Kit nach einem der Ansprüche 1 bis 3, **dadurch gekennzeichnet, dass** das Reagenz in fester Form vorliegt.

**12.** Kit nach Anspruch 11, **dadurch gekennzeichnet, dass** das Reagenz in Form einer Verreibung mit einem neutralen Trägerstoff vorliegt.

**13.** Kit nach einem der Ansprüche 1 bis 12, **dadurch gekennzeichnet, dass** es des weiteren mindestens eines der folgenden Elemente enthält: (a) ein Gefäß zur unmittelbaren Aufnahme des ausgeschiedenen Urins; (b) ein Pipettiermittel; (c) ein Analysegefäß, in dem die Urinprobe, das Reagenz und die Säure in Kontakt gebracht werden.

**14.** Kit nach einem der Ansprüche 1 bis 13, **dadurch gekennzeichnet, dass** es ein Analysegefäß enthält, in dem die Urinprobe, das Reagenz und die Säure in Kontakt gebracht werden, und dass die Farbskala mit dem Analysegefäß so verbunden ist, dass der Farbvergleich zwischen der Probelösung und der Farbskala unmittelbar visuell durchgeführt werden kann.

**15.** Kit nach Anspruch 1, **dadurch gekennzeichnet, dass** das Reagenz und die Säure beide in fester Form in einen Teststreifen eingearbeitet sind, der unmittelbar in die zu untersuchende Probe eingetaucht werden kann.

**16.** Kit nach Anspruch 15, **dadurch gekennzeichnet, dass** die Säure Citronensäure, Oxalsäure oder Trichloressigsäure ist.

**17.** Verfahren zur Bestimmung des Redox-Status im Urin, umfassend folgende Stufen:

(a) Vermischen einer zu untersuchenden Urinprobe mit

(i) mindestens einem Reagenz ausgewählt aus der Gruppe der Verbindungen der allgemeinen Formel (I)

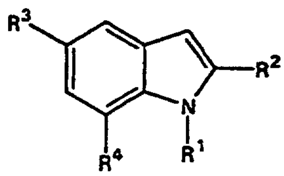

(I)

worin $R^1$ für H oder $C_1$-$C_{10}$-Alkyl steht, und $R^2$ für $C_1$-$C_{10}$-Alkyl steht, und

$R^3$ und $R^4$, unabhängig voneinander, für H, $C_1$-$C_{10}$-Alkyl, $C_6$-$C_{14}$-Aryl, $NH_2$, NHR, $NR_2$, NHCOR, OH, OR, OCOR, SH, SR, F, Cl, Br, $CF_3$ oder $CCl_3$ stehen, wobei Arylgruppen ggf. substituiert sind mit einem oder mehreren Substituenten ausgewählt aus der Gruppe bestehend aus $C_1$-$C_6$-Alkyl, $NH_2$, NHR, $NR_2$, NHCOR, OH, OR, OCOR, SH, SR, F, Cl, Br, $CF_3$ und $CCl_3$ und

wobei R jeweils für $C_1$-$C_6$-Alkyl steht und mehrere R-Gruppen ggf. unabhängig voneinander ausgewählt werden können,

und

(ii) mindestens einer Säure und

(b) nachdem sich eine stabile Färbung der Probe eingestellt hat, visuelles Vergleichen der Färbung der Probe mit einer Farbskala, wobei den einzelnen Farben der Skala ein bestimmter Redox-Status des zu untersuchenden Urins zugeordnet ist.

**18.** Verfahren nach Anspruch 17, **dadurch gekennzeichnet, dass** das Reagenz 2-Methylindol oder 1,2-Dimethylindol ist.

**19.** Verfahren nach Anspruch 17 oder 18, **dadurch gekennzeichnet, dass** die Säure Salzsäure ist.

**20.** Verfahren nach einem der Ansprüche 17 bis 19, **dadurch gekennzeichnet, dass** das Reagenz in Form einer Lösung in mindestens einem Lösungsmittel eingesetzt wird.

**21.** Verfahren nach Anspruch 20, **dadurch gekennzeichnet, dass** die Konzentration des Reagenz in der Lösung 0,1 bis 50 mmol/l beträgt.

**22.** Verfahren nach einem der Ansprüche 17 bis 21, **dadurch gekennzeichnet, dass** die Säure in Form einer Lösung in mindestens einem Lösungsmittel eingesetzt wird.

**23.** Verfahren nach Anspruch 22, **dadurch gekennzeichnet, dass** die Konzentration der Säure in der Lösung 0,1 bis 5,0 mol/l beträgt.

**24.** Verfahren nach einem der Ansprüche 20 bis 23, **dadurch gekennzeichnet, dass** das Lösungsmittel Isopropanol umfasst.

**25.** Verfahren nach einem der Ansprüche 17 bis 24, **dadurch gekennzeichnet, dass** das eingesetzte Reagenz oder eine Lösung davon zunächst in einem verschlossenen Behälter enthalten und von der Säure getrennt ist, der durch vorsätzliche mechanische Einwirkung zerstört oder geöffnet wird und so angeordnet ist, dass das Reagenz durch die Zerstörung oder Öffnung des Behälters unmittelbar in Kontakt mit der Säure und/oder mit einer Lösung davon und/oder mit der Urinprobe tritt.

**26.** Verfahren nach einem der Ansprüche 17 bis 24, **dadurch gekennzeichnet, dass** die eingesetzte Säure oder eine Lösung davon zunächst in einem verschlossenen Behälter enthalten und von dem Reagenz getrennt ist, der durch vorsätzliche mechanische Einwirkung zerstört oder geöffnet wird und so angeordnet ist, dass die Säure durch die Zerstörung oder Öffnung des Behälters unmittelbar in Kontakt mit dem Reagenz und/oder mit einer Lösung davon und/oder mit der Urinprobe tritt.

**27.** Verfahren nach Anspruch 17, **dadurch gekennzeichnet, dass** das Reagenz in fester Form eingesetzt wird.

**28.** Verfahren nach Anspruch 27, **dadurch gekennzeichnet, dass** das Reagenz in Form einer Verreibung mit einem neutralen Trägerstoff eingesetzt wird.

**29.** Verfahren nach Anspruch 17, **dadurch gekennzeichnet, dass** das Reagenz und die Säure beide in fester Form in einem Teststreifen eingesetzt werden, der unmittelbar in die zu untersuchende Probe eingetaucht wird.

**30.** Verfahren nach Anspruch 29, **dadurch gekennzeichnet, dass** die Säure Citronensäure, Oxalsäure oder Trichloressigsäure ist.

## Claims

**1.** Kit for determining redox status in urine, comprising:

(a) at least one reagent selected from the group of compounds with the general formula (I)

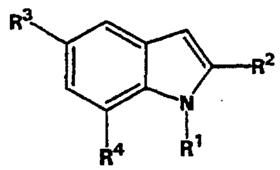

(I)

where $R^1$ denotes H or $C_1$-$C_{10}$-alkyl, $R^2$ denotes $C_1$-$C_{10}$-alkyl, and $R^3$ and $R^4$, independently, denote H, $C_1$-$C_{10}$-alkyl, $C_6$-$C_{14}$-aryl, $NH_2$, NHR, $NR_2$, NHCOR, OH, OR, OCOR, SH, SR, F, Cl, Br, $CF_3$ or $CCl_3$.,
where aryl groups are optionally substituted by one or more substituents selected from the group consisting of $C_1$-$C_6$-alkyl, $NH_2$, NHR, $NR_2$, NHCOR, OH, OR, OCOR, SH, SR, F, Cl, Br, $CF_3$ and $CCl_3$ and
where R denotes $C_1$-$C_6$-alkyl, and several R groups can optionally be selected independently,

(b) at least one acid and

(c) a colour scale for the visual colour comparison, where a certain redox status of the urine to be analysed is assigned to individual colours on the scale.

**2.** Kit according to claim 1, **characterised in that** the reagent is 2-methyl indole or 1,2-dimethyl indole.

**3.** Kit according to claim 1 or 2, **characterised in that** the acid is hydrochloric acid.

**4.** Kit according to one of claims 1 to 3, **characterised in that** the reagent is present in the form of a solution in at least one solvent.

**5.** Kit according to claim 4, **characterised in that** the concentration of the reagent in the solution is 0.1 to 50 mmol/l.

**6.** Kit according to one of claims 1 to 5, **characterised in that** the acid is present in the form of a solution in at least one solvent.

**7.** Kit according to claim 6, **characterised in that** the concentration of the acid in the solution is 0.1 to 5.0 mol/l.

**8.** Kit according to one of claims 4 to 7, **characterised in that** the solvent comprises isopropanol.

**9.** Kit according to one of claims 1 to 8, **characterised in that** the reagent or a solution of it is contained in a sealed container and is separate from the acid, which container can be easily destroyed or opened by deliberate mechanical action, and is arranged so that the reagent comes directly into contact with the acid and/or with a solution of it and/or with the urine sample as a result of the destruction or opening of the container.

**10.** Kit according to one of claims 1 to 8, **characterised in that** the acid or a solution of it is contained in a sealed container and is separate from the reagent, which container can easily be destroyed or opened by deliberate mechanical action, and is arranged so that the acid comes directly into contact with the reagent and/or with a solution of it and/or with the urine sample as a result of the destruction or opening of the container.

**11.** Kit according to one of claims 1 to 3, **characterised in that** the reagent is present in solid form.

**12.** Kit according to claim 11, **characterised in that** the reagent is present in the form of a mixture with a neutral carrier substance.

**13.** Kit according to one of claims 1 to 12, **characterised in that** it also contains at least one of the following elements: (a) a vessel for immediate receiving of the excreted urine; (b) a means of pipetting; (c) an analysis vessel in which the urine sample, the reagent and acid are brought into contact.

**14.** Kit according to one of claims 1 to 13, **characterised in that** it contains an analysis vessel in which the urine sample, the reagent and the acid are brought into contact, and **in that** the colour scale is connected to the analysis vessel so that the colour comparison between the sample solution and the colour scale can be carried out immediately and visually.

**15.** Kit according to claim 1, **characterised in that** the reagent and the acid are both incorporated into the test strip in solid form, which strip can be immersed directly in the sample to be analysed.

**16.** Kit according to claim 15, **characterised in that** the acid is citric acid, oxalic acid or trichloroacetic acid.

**17.** Method for determining the redox status in urine, comprising the following stages:

(a) mixing a urine sample to be analysed with

(i) at least one reagent selected from the group of compounds with the general formula (I)

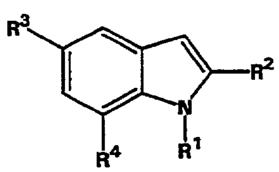

(I)

where $R^1$ denotes H or $C_1$-$C_{10}$-alkyl, $R^2$ denotes $C_1$-$C_{10}$-alkyl, and $R^3$ and $R^4$, independently, denote H, $C_1$-$C_{10}$-alkyl, $C_6$-$C_{14}$-aryl, $NH_2$, NHR, $NR_2$, NHCOR, OH, OR, OCOR, SH, SR, F, Cl, Br, $CF_3$ or $CCl_3$, where aryl groups are optionally substituted by one or more substituents selected from the group consisting of $C_1$-$C_6$-alkyl, $NH_2$, NHR, $NR_2$, NHCOR, OH, OR, OCOR, SH, SR, F, Cl, Br, $CF_3$ and $CCl_3$ and where R in each case denotes $C_1$-$C_6$-alkyl, and several R groups can optionally be selected independently, and

(ii) at least one acid and

(b) after a stable coloration of the sample has been achieved, visual comparison of the coloration of the sample with a colour scale, a specific redox status of the urine to be analysed being assigned to the individual colours of the scale.

**18.** Method according to claim 17, **characterised in that** the reagent is 2-methyl indole or 1,2-dimethyl indole.

**19.** Method according to claim 17 or 18, **characterised in that** the acid is hydrochloric acid.

**20.** Method according to one of claims 17 to 19, **characterised in that** the reagent is used in the form of a solution in at least one solvent.

**21.** Method according to claim 20, **characterised in that** the concentration of the reagent in the solution is 0.1 to 50 mmol/l.

**22.** Method according to one of claims 17 to 21, **characterised in that** the acid is used in the form of a solution in at least one solvent.

**23.** Method according to claim 22, **characterised in that** the concentration of the acid in the solution is 0.1 to 5.0 mol/l.

**24.** Method according to one of claims 20 to 23, **characterised in that** the solvent comprises isopropanol.

**25.** Method according to one of claims 17 to 24, **characterised in that** the reagent used or a solution of it is first contained in a sealed container and separate from the acid, which container is destroyed or opened by deliberate mechanical action and is arranged so that the reagent immediately comes into contact with the acid and/or with a solution of it and/or with the urine sample as a result of the destruction or opening of the container.

**26.** Method according to one of claims 17 to 24, **characterised in that** the acid used or a solution of it is first contained in a sealed container and is separate from the reagent, which container is destroyed or opened by deliberate mechanical action and is arranged so that the acid immediately comes into contact with the reagent and/or with a solution of it and/or with the urine sample as a result of the destruction or opening of the container.

**27.** Method according to claim 17, **characterised in that** the reagent is used in solid form.

**28.** Method according to claim 27, **characterised in that** the reagent is used in the form of a mixture with a neutral carrier substance.

**29.** Method according to claim 17, **characterised in that** the reagent and the acid are both used in solid form in a test strip which is immersed directly in the sample to be analysed.

**30.** Method according to claim 29, **characterised in that** the acid is citric acid, oxalic acid or trichloroacetic acid.

**Revendications**

**1.** Kit pour la détermination de l'état rédox de l'urine, comprenant :

(a) au moins un réactif choisi parmi le groupe des composés de la formule générale (I) :

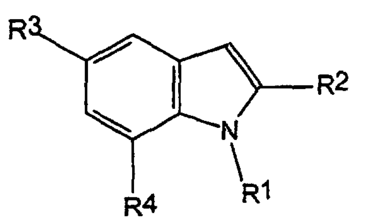

(I)

où $R^1$ représente H ou un alkyle en $C_1$-$C_{10}$, $R^2$ représente un alkyle en $C_1$-$C_{10}$, et $R^3$ et $R^4$ représentent indépendamment l'un de l'autre, H, un alkyle en $C_1$-$C_{10}$, un aryle en $C_6$-$C_{14}$, $NH_2$, NHR, $NR_2$, NHCOR, OH, OR, OCOR, SH, SR, F, Cl, Br, $CF_3$ ou $CCl_3$, où les radicaux aryle sont le cas échéant substitués avec un ou plusieurs substituants choisis parmi le groupe consistant en un alkyle en $C_1$-$C_6$, $NH_2$, NHR, $NR_2$, NHCOR, OH, OR, OCOR, SH, SR, F, Cl, Br, $CF_3$ et $CCl_3$, et où R représente chaque fois, un alkyle en $C_1$-$C_6$, et plusieurs radicaux R peuvent être le cas échéant, choisis indépendamment l'un de l'autre ;

(b) au moins un acide, et

(c) une échelle de couleur pour la comparaison visuelle des couleurs, où chaque couleur de l'échelle est attribuée à un état rédox déterminé de l'urine à examiner.

**2.** Kit selon la revendication 1, **caractérisé en ce que** le réactif est le 2-méthylindole ou le 1,2-diméthylindole.

**3.** Kit selon la revendication 1 ou 2, **caractérisé en ce que** l'acide est l'acide chlorhydrique.

**4.** Kit selon une quelconque des revendications 1 à 3, **caractérisé en ce que** le réactif est présent sous la forme d'une solution dans au moins un solvant.

**5.** Kit selon la revendication 4, **caractérisé en ce que** la concentration du réactif dans la solution se situe dans l'intervalle allant de 0,1 à 50 mmoles/litre.

**6.** Kit selon une quelconque des revendications 1 à 5, **caractérisé en ce que** l'acide est présent sous la forme d'une solution dans au moins un solvant.

**7.** Kit selon la revendication 6, **caractérisé en ce que** la concentration de l'acide dans la solution se situe dans l'intervalle allant de 0,1 à 5,0 mmoles/litre.

**8.** Kit selon une quelconque des revendications 4 à 7, **caractérisé en ce que** le solvant comprend l'isopropanol.

**9.** Kit selon une quelconque des revendications 1 à 8, **caractérisé en ce que** le réactif ou une solution de celui-ci est contenu dans un récipient fermé et séparé de l'acide, qui peut être facilement détruit ou ouvert par action mécanique et est disposé de sorte que le réactif entre directement en contact avec l'acide et/ou avec une solution de celui-ci et/ou avec l'échantillon d'urine par la destruction ou l'ouverture du récipient.

**10.** Kit selon une quelconque des revendications 1 à 8, **caractérisé en ce que** l'acide ou une solution de celui-ci est contenu dans un récipient fermé et séparé du réactif, qui peut être facilement détruit ou ouvert par action mécanique et est disposé de sorte que l'acide entre directement en contact avec le réactif et/ou avec une solution de celui-ci et/ou avec l'échantillon d'urine par la destruction ou l'ouverture du récipient.

**11.** Kit selon une quelconque des revendications 1 à 3, **caractérisé en ce que** le réactif se trouve sous forme solide.

**12.** Kit selon la revendication 11, **caractérisé en ce que** le réactif est présent sous forme d'un malaxage avec une substance support neutre.

**13.** Kit selon une quelconque des revendications 1 à 12, **caractérisé en ce qu'**il contient en outre, au moins un des éléments suivants : (a) un récipient pour la réception directe de l'urine séparée ; (b) un dispositif de pipetage ; (c) un récipient d'analyse, dans lequel l'échantillon d'urine, le réactif et l'acide sont mis en contact.

**14.** Kit selon une quelconque des revendications 1 à 13, **caractérisé en ce qu'**il contient un récipient d'analyse, dans lequel l'échantillon d'urine, le réactif et l'acide sont mis en contact, et **en ce que** l'échelle de couleur est reliée au récipient d'analyse, de sorte que la comparaison des couleurs entre la solution d'échantillon et l'échelle de couleur peut être directement réalisée visuellement.

**15.** Kit selon la revendication 1, **caractérisé en ce que** le réactif et l'acide sont tous deux traités sous forme solide en une bande de test, qui peut être directement immergée dans l'échantillon à analyser.

**16.** Kit selon la revendication 15, **caractérisé en ce que** l'acide est l'acide citrique, l'acide oxalique ou l'acide trichloroacétique.

**17.** Procédé de détermination de l'état rédox de l'urine, comprenant les étapes suivantes :

(a) mélange d'un échantillon d'urine à analyser, avec

(i) au moins un réactif choisi parmi le groupe des composés de la formule générale (I) :

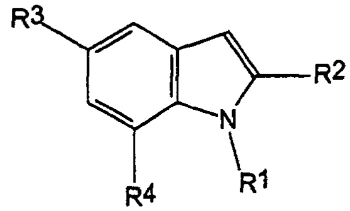

(I)

où $R^1$ représente H ou un alkyle en $C_1$-$C_{10}$, $R^2$ représente un alkyle en $C_1$-$C_{10}$, et $R^3$ et $R^4$ représentent indépendamment l'un de l'autre, H, un alkyle en $C_1$-$C_{10}$, un aryle en $C_6$-$C_{14}$, $NH_2$, NHR, $NR_2$, NHCOR, OH, OR, OCOR, SH, SR, F, Cl, Br, $CF_3$ ou $CCl_3$, où les radicaux aryle sont le cas échéant substitués avec un ou plusieurs substituants choisis parmi le groupe consistant en un alkyle en $C_1$-$C_6$, $NH_2$, NHR, $NR_2$, NHCOR, OH, OR, OCOR, SH, SR, F, Cl, Br, $CF_3$ et $CCl_3$, et où R représente chaque fois, un alkyle en $C_1$-$C_6$, et plusieurs radicaux R peuvent être le cas échéant, choisis indépendamment l'un de l'autre ;

(ii) au moins un acide, et

(b) après qu'une couleur stable de l'échantillon se soit ajustée, comparaison visuelle de la couleur de l'échantillon avec une échelle de couleur, où chaque couleur de l'échelle est attribuée à un état rédox déterminé de l'urine à analyser.

**18.** Procédé selon la revendication 17, **caractérisé en ce que** le réactif est le 2-méthylindole ou le 1,2-diméthylindole.

**19.** Procédé selon la revendication 17 ou 18, **caractérisé en ce que** l'acide est l'acide chlorhydrique.

**20.** Procédé selon une quelconque des revendications 17 à 19, **caractérisé en ce que** le réactif est présent sous la forme d'une solution dans au moins un solvant.

**21.** Procédé selon la revendication 20, **caractérisé en ce que** la concentration du réactif dans la solution se situe dans l'intervalle allant de 0,1 à 50 mmoles/litre.

**22.** Procédé selon une quelconque des revendications 17 à 21, **caractérisé en ce que** l'acide est présent sous la forme d'une solution dans au moins un solvant.

**23.** Procédé selon la revendication 22, **caractérisé en ce que** la concentration de l'acide dans la solution se situe dans l'intervalle allant de 0,1 à 5,0 mmoles/litre.

**24.** Procédé selon une quelconque des revendications 20 à 23, **caractérisé en ce que** le solvant comprend l'isopropanol.

**25.** Procédé selon une quelconque des revendications 17 à 24, **caractérisé en ce que** le réactif ou une solution de celui-ci est contenu dans un récipient fermé et séparé de l'acide, qui peut être facilement détruit ou ouvert par action mécanique et est disposé de sorte que le réactif entre directement en contact avec l'acide et/ou avec une solution de celui-ci et/ou avec l'échantillon d'urine par la destruction ou l'ouverture du récipient.

**26.** Procédé selon une quelconque des revendications 17 à 24, **caractérisé en ce que** l'acide ou une solution de celui-ci est contenu dans un récipient fermé et séparé du réactif, qui peut être facilement détruit ou ouvert par action mécanique et est disposé de sorte que l'acide entre directement en contact avec le réactif et/ou avec une solution de celui-ci et/ou avec l'échantillon d'urine par la destruction ou l'ouverture du récipient.

**27.** Procédé selon la revendication 17, **caractérisé en ce que** le réactif se trouve sous forme solide.

**28.** Procédé selon la revendication 27, **caractérisé en ce que** le réactif est présent sous forme d'un malaxage avec une substance support neutre.

**29.** Procédé selon la revendication 17, **caractérisé en ce que** le réactif et l'acide sont tous deux mis en oeuvre sous forme solide en une bande de test, qui peut être directement immergée dans l'échantillon à analyser.

**30.** Procédé selon la revendication 29, **caractérisé en ce que** l'acide est l'acide citrique, l'acide oxalique ou l'acide trichloroacétique.

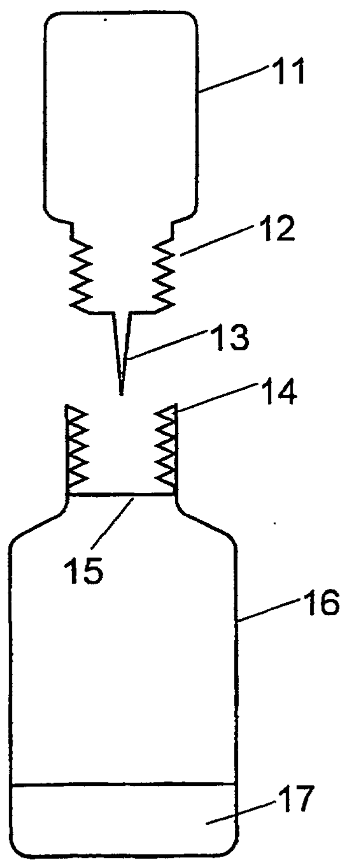


Fig. 1

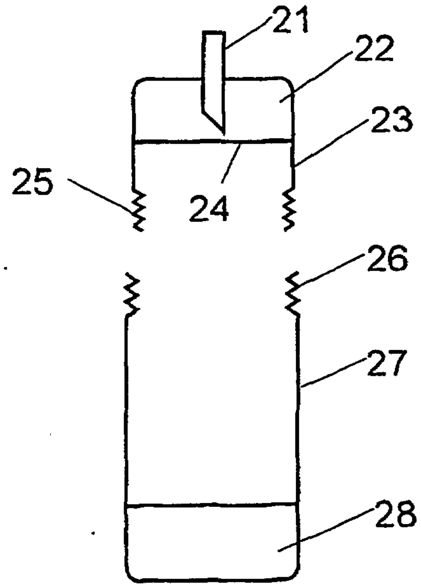


Fig. 2

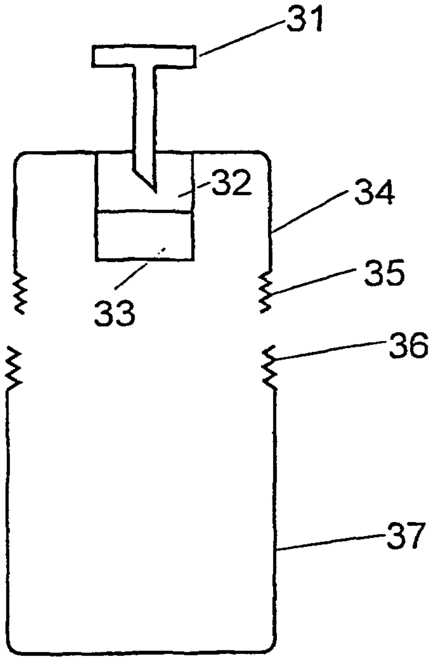


Fig. 3

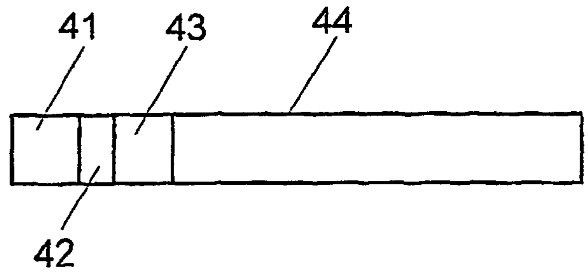


Fig. 4

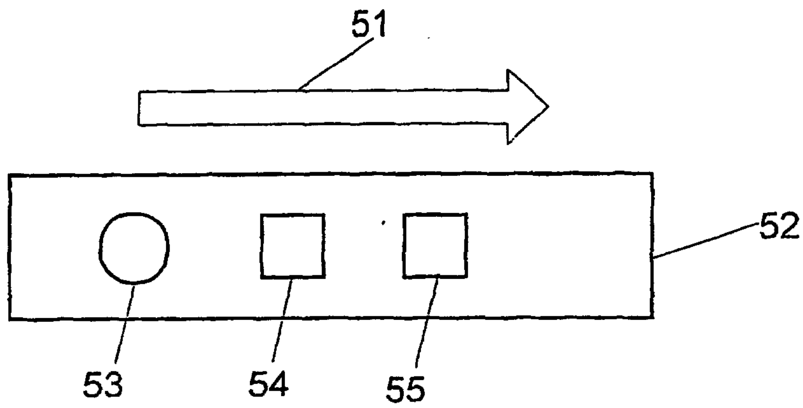


Fig. 5

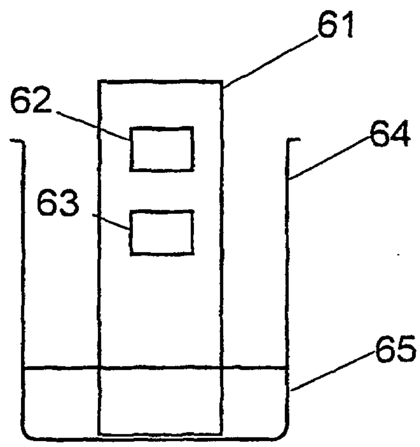


Fig. 6